(19) Europäisches Patentamt
European Patent Office
Office européen des brevets

(11) **EP 3 797 698 B1**

(12) **EUROPÄISCHE PATENTSCHRIFT**

(45) Veröffentlichungstag und Bekanntmachung des Hinweises auf die Patenterteilung:
**06.11.2024 Patentblatt 2024/45**

(21) Anmeldenummer: **19200330.9**

(22) Anmeldetag: **30.09.2019**

(51) Internationale Patentklassifikation (IPC):
**A61B 6/02** (2006.01) **A61B 6/00** (2024.01)
**G06T 11/00** (2006.01)

(52) Gemeinsame Patentklassifikation (CPC):
**A61B 6/025; A61B 6/481; A61B 6/482;
A61B 6/502; A61B 6/5217; A61B 6/5223;
G06T 11/003**

(54) **VERFAHREN ZUM ERZEUGEN EINES SYNTHETISCHEN MAMMOGRAMMS BASIEREND AUF EINER DUAL-ENERGY-TOMOSYNTHESEAUFNAHME**

METHOD FOR GENERATING A SYNTHETIC MAMMOGRAM BASED ON DUAL-ENERGY TOMOSYNTHESIS CAPTURE

PROCÉDÉ DE GÉNÉRATION D'UN MAMMOGRAMME SYNTHÉTIQUE SE BASANT SUR UNE IMAGERIE PAR TOMOSYNTHÈSE DOUBLE ÉNERGIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**31.03.2021 Patentblatt 2021/13**

(73) Patentinhaber: **Siemens Healthineers AG
91301 Forchheim (DE)**

(72) Erfinder:
• **Hörnig, Mathias
91096 Möhrendorf (DE)**
• **Kappler, Steffen
91090 Effeltrich (DE)**
• **Wicklein, Julia
91077 Neunkirchen a. Br. (DE)**

(74) Vertreter: **Siemens Healthineers
Patent Attorneys
Postfach 22 16 34
80506 München (DE)**

(56) Entgegenhaltungen:
**US-A1- 2014 072 096 US-A1- 2017 011 534**

• **HAILIANG HUANG ET AL: "Comparison of contrast-enhanced digital mammography and contrast-enhanced digital breast tomosynthesis for lesion assessment", JOURNAL OF MEDICAL IMAGING, vol. 6, no. 03, 13 February 2019 (2019-02-13), 1000 20th St. Bellingham WA 98225-6705 USA, pages 1, XP055671269, ISSN: 2329-4302, DOI: 10.1117/1.JMI.6.3.031407**
• **VAN SCHIE GUIDO ET AL: "Generating Synthetic Mammograms From Reconstructed Tomosynthesis Volumes", IEEE TRANSACTIONS ON MEDICAL IMAGING, IEEE SERVICE CENTER, PISCATAWAY, NJ, US, vol. 32, no. 12, 1 December 2013 (2013-12-01), pages 2322 - 2331, XP011532169, ISSN: 0278-0062, [retrieved on 20131125], DOI: 10.1109/TMI.2013.2281738**

**Beschreibung**

Die Erfindung betrifft ein Verfahren zum Erzeugen eines synthetischen Mammogramms basierend auf einer Dual-Energy-Tomosyntheseaufnahme eines Untersuchungsbereichs und ein Mammographiesystem dazu, welche eine Berechnung eines synthetischen Mammogramms mit einer reduzierten Überlagerung tumorbehafteter Bereiche ermöglichen.

Die Kontrastmittel verstärkte Dual-Energy-Mammographie der Brust (CEDEM / Contrast Enhanced Dual Energy Mammography, CEDM / Contrast Enhanced Digital Mammography) ist eine Methode in der Röntgendiagnostik, bei der hochenergetische (Abkürzung: HE) Röntgenbilder/-aufnahmen nach Kontrastmittelgabe erzeugt und Niedrigenergiebilder/-aufnahmen (Abkürzung: LE) ohne Kontrastmitteldarstellung von ihnen gewichtet subtrahiert werden. Ziel ist die bessere Erkennbarkeit von Läsionen durch die Darstellung der Kontrastmittelanreicherung innerhalb der an die Tumore zufließenden Blutgefäße. Von der Dual-Energy-Bildgebung in der Digitalen Mammographie (DM) unter Verwendung von Jod als Kontrastmittel, auch bekannt als Zwei-Spektren-Methode, wird eine verbesserte Diagnose für den Radiologen erwartet sowie eine Verbesserung der Sensitivität und Spezifität. CEDEM kann beispielsweise in Form von einer sogenannten Titanium Contrast Enhanced Mammography durchgeführt werden.

Die Adaption obigen Verfahrens von 2D auf 3D wird als CEDET (Contrast Enhanced Dual Energy Tomosynthesis) bezeichnet. Dabei werden zwei Tomosynthesescans mit unterschiedlichen Röntgenspektren bzw. unterschiedlichen Röhrenspannungen aufgenommen, bei denen analog zu CEDEM die Darstellung einer Jod-Kontrastmittelanreicherung erfolgt. Das resultierende Subtraktionsvolumen basierend auf den beiden Tomosynthesescans entspricht einer 3D-Karte bzw. 3D-Verteilung der Tumor relevanten Bereiche. Im Rahmen einer digitalen Tomosyntheseaufnahme (engl.: Digital Breast Tomosynthesis, DBT) wird üblicherweise zusätzlich zu den (dreidimensionalen) Schichtbildern ein synthetisches Mammogramm berechnet, das einer zweidimensionalen Aufnahme des vorhandenen Datensatzes entspricht und aus den Schichten rekonstruiert wird.

Aus der Druckschrift US 9 808 215 B2 ist beispielsweise ein Verfahren zum Berechnen eines synthetischen Mammogramms bekannt. Ein 2D-Mammogrammbild wird dabei aus mindestens einem von Tomosyntheseprojektionsbildern und/oder den von der Tomosynthese rekonstruierten Bilddaten synthetisiert. In einfachster Form kann die Mammographie durch Auswahl eines der Tomosyntheseprojektionsbilder für die Darstellung als synthetisierte Mammographie synthetisiert werden. Andere Methoden zur Synthese einer Mammographie beinhalten die Neuprojektion und Filterung von Projektionsdaten und/oder rekonstruierten Daten. Die synthetisierte Mammographie wird zusammen mit mindestens einem Teil der rekonstruierten Daten angezeigt, um die Überprüfung der rekonstruierten Daten zu erleichtern. Es wird ein vertrautes Bild erzeugt, das verwendet werden kann, um die Überprüfung eines Tomosynthese-Datensatzes zu erleichtern.

Aus der Publikation HAILIANG HUANG ET AL: "Comparison of contrast-enhanced digital mammography and contrast-enhanced digital breast tomosynthesis for lesion assessment", JOURNAL OF MEDICAL IMAGING, Bd. 6, Nr. 03, 13. Februar 2019 (2019-02-13), Seite 1, XP055671269, 1000 20th St. Bellingham WA 98225-6705 USA ISSN: 2329-4302, DOI: 10.1117/1.JMI.6.3.031407 ; ist bekannt, dass die kontrastverstärkte digitale Mammographie (CEDM) die Neovaskulatur von Brustläsionen in einer zweidimensionalen Kontrastverstärkungskarte zeigt. Die kontrastverstärkte digitale Brusttomosynthese (CEDBT) bietet eine dreidimensionale Kontrastverstärkung, die die Charakterisierung und Lokalisierung von Läsionen verbessern kann. Synthetisches CEDM kann aus CEDBT-Daten generiert werden und bietet eine mit CEDM vergleichbare Läsionskontrastverstärkung.

Aus der Druckschrift US 2017/011534 A1 ist ein Verfahren zur Erzeugung eines synthetischen zweidimensionalen Mammogramms mit erhöhtem Kontrast für Strukturen von Interesse bekannt, welches die Erfassung eines dreidimensionalen digitalen Brust-Tomosynthese-Volumens mit einer Vielzahl von Voxeln umfasst. Es wird eine dreidimensionale Relevanzkarte erzeugt, die für die Voxel die Relevanz der zugrunde liegenden Struktur für eine Diagnose kodiert. Ein synthetisches zweidimensionales Mammogramm wird auf der Grundlage des dreidimensionalen digitalen Brust-Tomosynthese-Volumens und der dreidimensionalen Relevanzkarte berechnet.

Bei einer üblichen Berechnung eines synthetischen Mammogramms basierend auf einer Durchschnittsintensitätsprojektion (engl.: average intensity projection, AIP) und einer Maximumsintensitätsprojektion (engl.: maximum intensity projection, MIP) basierend auf den Tomosyntheseschichten werden nicht alle tumorrelevanten Bereiche ausreichend deutlich dargestellt. So kann es beispielsweise durch Überlagerungen dichten Gewebes zum Verlust der Sichtbarkeit der einen Tumor begrenzenden Strukturen, beispielsweise sogenannter spiculi, und der Ränder (engl.: margins) kommen. Die Darstellung solcher Strukturen ist jedoch für die Diagnose besonders relevant und interessant, da sie das Erkennen eines Tumors für den befundenden Radiologen erleichtert und einen Indikator für die Malignität des Tumors darstellt.

Bei der herkömmlichen Tomosynthese, beispielsweise im Rahmen eines Screenings werden, auf künstlicher Intelligenz (AI) basierte CAD-Verfahren zur Erkennung von Läsionen innerhalb der 3D-Schichten herangezogen. Aus der Druckschrift EP 3 326 535 A1 ist beispielsweise ein Anzeigesystem zum Anzeigen von Daten der digitalen Brusttomosynthese (DBT) bekannt. Erste und zweite DBT-Volumenbilder der linken Brust einer Frau und erste und zweite DBT-Volumenbilder der rechten Brust der Frau werden durch eine Bilderzeugungseinheit bereitgestellt. Darüber hinaus wird für jedes DBT-Volumenbild ein zweidimensionales Navigationsbild durch eine Navigationsbildbereitstellungseinheit bereitgestellt, wobei einem Benutzer erlaubt ist, eine Position im Navigationsbild unter Verwendung einer Benutzeroberfläche anzugeben, woraufhin in einem DBT-Volumenbild einer Brust ein dem Standort zugeordneter CAD-Marker bestimmt wird und ein entsprechender CAD-Marker, falls vorhanden, in einem anderen DBT-Volumenbild der Brust bestimmt wird. Schnitte dieser DBT-Volumenbilder, die den CAD-Markern zugeordnet sind, werden auf einem Display angezeigt. Diese CAD-Verfahren beruhen auf Deep Learning Verfahren, bei welchen Neuronale Netze wie beispielsweise das U-Net mit einer Vielzahl an Datensätzen mit bekannten Läsionspositionen, insbesondere mit Bildannotationen, trainiert werden. Anschließend sind die trainierten Netze in der Lage, auf unbekannten Datensätzen eine Wahrscheinlichkeitsverteilung der Tumor relevanten Bereiche zu erzeugen.

Die daraus entstehenden sogenannten Probability Maps oder Wahrscheinlichkeitskarten können anschließend verwendet werden, um die erkannten Bereiche im synthetischen zweidimensionalen Bild sichtbar zu machen. Bei der Erzeugung erfolgt durch eine unterschiedliche Gewichtung der einzelnen Schichten anhand der Wahrscheinlichkeitsverteilungen bzw. Wahrscheinlichkeitskarte und verhindert oder reduziert somit störende Überlagerungen von Tumor behafteten Bereichen. Aus der Druckschrift US 10 010 302 B2 ist bekannt, dass ein 2D-Mammogrammbild aus mindestens einem von Tomosyntheseprojektionsbildern und/oder den von der Tomosynthese rekonstruierten Bilddaten synthetisiert wird. In einfachster Form kann die Mammographie durch Auswahl eines der Tomosyntheseprojektionsbilder für die Darstellung als synthetisierte Mammographie synthetisiert werden. Andere Methoden zur Synthese einer Mammographie beinhalten die Neuprojektion und Filterung von Projektionsdaten und/oder rekonstruierten Daten. Die synthetisierte Mammographie wird zusammen mit mindestens einem Teil der rekonstruierten Daten angezeigt, um die Überprüfung der rekonstruierten Daten zu erleichtern. Damit kann ein vertrautes Bild erzeugt werden, das verwendet werden kann, um die Überprüfung eines Tomosynthese-Datensatzes zu erleichtern.

Es ist Aufgabe der Erfindung, ein Verfahren zum Erzeugen eines synthetischen Mammogramms, ein Mammographiesystem, ein Computerprogrammprodukt und ein computerlesbares Medium anzugeben, welche eine vereinfachte und alternative Berechnung eines synthetischen Mammogramms mit einer reduzierten Überlagerung tumorbehafteter Bereiche ermöglichen.

Die Aufgabe wird erfindungsgemäß gelöst durch ein Verfahren zum Erzeugen eines synthetischen Mammogramms basierend auf einer Dual-Energy-Tomosyntheseaufnahme nach Anspruch 1, ein Mammographiesystem nach Anspruch 12, ein Computerprogrammprodukt nach Anspruch 13 und ein computerlesbares Medium nach Anspruch 14.

Die Erfindung betrifft ein Verfahren zum Erzeugen eines synthetischen Mammogramms, insbesondere mit einer reduzierten Überlagerung tumorbehafteter Bereiche, basierend auf einer Dual-Energy-Tomosyntheseaufnahme eines Untersuchungsbereichs. Das Verfahren weist die Schritte des Aufnehmens einer Niedrigenergietomosyntheseaufnahme, des Aufnehmens einer Hochenergietomosyntheseaufnahme, des Ermittelns, des Erstellens, des Erzeugens und des Anzeigens auf. Im Schritt des Aufnehmens einer Niedrigenergietomosyntheseaufnahme wird eine Niedrigenergietomosyntheseaufnahme mit einem ersten Röntgenenergiespektrum aufgenommen. Im Schritt des Aufnehmens einer Hochenergietomosyntheseaufnahme wird eine Hochenergietomosyntheseaufnahme mit einem zweiten gegenüber dem ersten Röntgenenergiespektrum höherenergetischen Röntgenenergiespektrum aufgenommen, wobei der Untersuchungsbereich eine Kontrastmittelverteilung aufweist. Im Schritt des Ermittelns wird ein Subtraktionsvolumen basierend auf der Hochenergietomosyntheseaufnahme und der Niedrigenergietomosyntheseaufnahme ermittelt. Im Schritt des Erstellens wird eine dreidimensionale Wahrscheinlichkeitskarte mit einem Wichtungsfaktor pro Voxel basierend auf dem Subtraktionsvolumen erstellt. Im Schritt des Erzeugens wird ein synthetisches Mammogramm basierend auf der dreidimensionalen Wahrscheinlichkeitskarte, und insbesondere der Niedrigenergietomosyntheseaufnahme, erzeugt. Das synthetische Mammogramm kann als kontrastverstärktes synthetisches Mammogramm bezeichnet werden.

Zur Vorbereitung der Aufnahme wird der Patientin bzw. dem Patienten ein Kontrastmittel verabreicht. Während einer etwaigen Wartezeit, beispielsweise zwischen 1,5 und 2 Minuten, beginnt sich das Kontrastmittel insbesondere im Untersuchungsbereich zu verteilen. Die Patientin kann nun positioniert und die Brust komprimiert werden. Zunächst kann eine Niedrigenergietomosyntheseaufnahme, beispielsweise mit einer Wolfram-Anode mit Rhodium-Filter bei einer Röhrenspannung zwischen 24 und 32 kV, aufgenommen werden. In der Niedrigenergietomosyntheseaufnahme ist das Kontrastmittel bereits im Gewebe zumindest teilweise verteilt, jedoch ist der Einfluss des Kontrastmittels auf die Niedrigenergietomosyntheseaufnahme gering. Anschließend kann die Hochenergietomosyntheseaufnahme, beispielsweise mit einem Titan-Filter und einer Röhrenspannung von beispielsweise 49 kV, aufgenommen werden. In der Hochenergietomosyntheseaufnahme ist das Kontrastmittel im Gewebe verteilt, der Einfluss des Kontrastmittels auf die Hochenergietomosyntheseaufnahme ist deutlich erkennbar.

Die Hochenergietomosyntheseaufnahme wird aufgenommen, nachdem ein Kontrastmittel in den Untersuchungsbereich eingebracht wurde. Während der Hochenergietomosyntheseaufnahme liegt eine Kontrastmittelanreicherung, insbesondere eine Jodanreicherung, im Untersuchungsbereich vor. Während der Niedrigenergietomosyntheseaufnahme kann dagegen eine Kontrastmittelanreicherung oder keine Kontrastmittelanreicherung im Untersuchungsbereich vorhanden sein. Üblicherweise, insbesondere um den Patientenkomfort zu erhöhen, kann sowohl in der Niedrigenergietomosyntheseaufnahme und in der Hochenergietomosyntheseaufnahme die Kontrastmittelanreicherung vorhanden sein. Der Untersuchungsbereich kann während des Aufnehmens der Niedrigenergietomosyntheseaufnahme eine Kontrastmittelverteilung aufweisen. Die Hochenergietomosyntheseaufnahme und die Niedrigenergietomosyntheseaufnahme umfassen jeweils ein Datensatz mit einer Mehrzahl von Projektionsdatensätzen aufgenommen unter einer Mehrzahl von Projektionswinkeln. Davon ausgehend kann jeweils eine Tomosynthesevolumen für die Hochenergietomosyntheseaufnahme und die Niedrigenergietomosyntheseaufnahme rekonstruiert werden.

Beim Erzeugen kann insbesondere eine unterschiedliche Gewichtung der einzelnen Schichten bzw. Voxel anhand Wahrscheinlichkeitsverteilung der dreidimensionalen Wahrscheinlichkeitskarte vorgenommen werden. Es kann insbesondere die Niedrigenergietomosyntheseaufnahme mit der dreidimensionalen Wahrscheinlichkeitskarte zu einem kontrastverstärkten synthetischen Mammogramm kombiniert werden.

Das Subtraktionsvolumen kann basierend auf dem rekonstruierten Tomosynthesevolumen der Hochenergietomosyntheseaufnahme und der Niedrigenergietomosyntheseaufnahme berechnet werden. Dabei werden die Bildwerte jeweils entsprechender Voxel des Tomosynthesevolumens der Hochenergietomosyntheseaufnahme (HE) und des Tomosynthesevolumens der Niedrigenergietomosyntheseaufnahme (LE) gewichtet subtrahiert. Beispielsweise kann die Subtraktion nach folgender Gleichung durchgeführt werden:

$$\ln(x) = \ln(HE) - w \cdot \ln(LE) \, .$$

Der Faktor w kann in Abhängigkeit der Brustdicke/-dichte, beispielsweise basierend auf der Kompressionsdicke, gewählt werden. Auf dem Wert x kann der Wichtungsfaktor der dreidimensionalen Wahrscheinlichkeitskarte basieren. Der Wertebereich der Wichtungsfaktoren der dreidimensionalen Wahrscheinlichkeitskarte kann sich bevorzugt von 0 bis 200 oder von 0 bis 2 erstrecken. Der Wichtungsfaktor gibt für einen Voxel eine Wahrscheinlichkeit bzw. ein Gewicht an. Der Wichtungsfaktor der dreidimensionalen Wahrscheinlichkeitskarte kann eine Kontrastmittelkonzentration umfassen, welche basierend auf dem Wert x bestimmt werden kann. Alternativ kann der Wichtungsfaktor diskrete Werte annehmen, beispielsweise 0 für einen Hintergrund, 1 für Brustgewebe und 2 für eine Läsion.

Die dreidimensionale Wahrscheinlichkeitskarte bzw. die dreidimensionale Probability Map wird im Schritt des Erstellens basierend auf dem Subtraktionsvolumen erstellt. Die Wichtungsfaktoren der dreidimensionalen Wahrscheinlichkeitskarte können ein Maß für die Kontrastmittelkonzentration, insbesondere Jodkonzentration, sein.

Die Erfinder schlagen einen neuen, alternativen Ansatz für eine Probability Map bzw. Wahrscheinlichkeitskarte für den Fall einer kontrastmittelverstärkten Tomosynthese Aufnahme vor. Anstatt eines auf Deep Learning basierten Verfahrens mit entsprechenden Trainingsdaten basiert die Probability Map bzw. Wahrscheinlichkeitskarte erfindungsgemäß auf der durch Jodanreicherung gewonnenen 3D-Karte bzw. 3D-Verteilung des Suktraktionsvolumens. Dabei entspricht die Stärke des angereicherten Jodanteils der Wahrscheinlichkeit bzw. dem Grad der Malignität der Läsion, aufgrund derer eine Jodanreicherung erfolgt ist. In einer Ausführungsform können die Daten einer quantitativen Analyse der Jodkonzentration aus den CEDET-Daten (in mg Jod/cm$^3$) hierfür genutzt werden. Anschließend kann ebenfalls eine überlagerungsfreie oder mindestens überlagerungsreduzierte Darstellung der, insbesondere für die Diagnose, relevanten Bereiche innerhalb des synthetischen Mammogramms erfolgen.

Bei einer kontrastmittelverstärkten Brusttomosynthese kann das entsprechende ohnehin vorhandene Kontrastmittelbild, insbesondere Jodbild bzw. Subtraktionsbild bzw. -bild, vorteilhaft zusätzlich zur Erstellung einer dreidimensionalen Wahrscheinlichkeitskarte genutzt werden. Vorteilhaft kann ein aufwendiges Sammeln annotierter Daten für ein CAD-basiertes Verfahren vermieden werden. Das Sammeln annotierter Daten wäre für einen Ansatz mittels Neuronaler Netze notwendig. Da die kontrastmittelverstärkte Dual-Energy-Tomosyntheseaufnahme eine neue Art der Röntgendiagnose zur Tumorerkennung darstellt, kann derzeit noch nicht auf eine ausreichende Datenbasis zurückgegriffen werden. Die Ausgabequalität solcher trainierten Netze hängt jedoch weitestgehend von Anzahl und Qualität der annotierten Trainingsdaten ab. Vorteilhaft kann für die kontrastmittelverstärkte Dual-Energy-Tomosyntheseaufnahme eine leichter verfügbare Methode zur Berechnung einer dreidimensionalen Wahrscheinlichkeitskarte ermöglicht werden.

Gemäß einem Aspekt der Erfindung wird im Schritt des Erzeugens ein erstes synthetisches Mammogramm basierend auf der Niedrigenergietomosyntheseaufnahme erstellt. Das erste synthetische Mammogramm kann basierend auf der Niedrigenergietomosyntheseaufnahme erzeugt werden, beispielsweise mittels bekannter Verfahren zum Erzeugen eines synthetischen Mammogramms.

Das erste synthetische Mammogramm bzw. ein synthetisches Mammogramm kann im Allgemeinen nachfolgende Schritte umfassen und insbesondere auf der Niedrigenergietomosyntheseaufnahme mit einer Mehrzahl von Projektionsdatensätzen basieren. Bevorzugt wird zumindest ein (erstes) synthetisches Mammogramm entsprechend des mittleren Projektionsdatensatzes, insbesondere bei einem Projektionswinkel von 0 Grad, erzeugt. Der mittlere Projektionswinkel kann dabei insbesondere den Winkel bezeichnen, bei dem der Zentralstrahl der Röntgenquelle im Wesentlichen senkrecht auf die komprimierte Brust bzw. senkrecht auf das obere Kompressionselement einfällt. Ein (erstes) synthetisches Mammogramm kann jeweils einem Projektionswinkel zugeordnet werden. Es kann für einen Projektionswinkel ein (erstes) synthetisches Mammogramm erzeugt werden. Das (erstes) synthetische Mammogramm kann insbesondere auf der Projektionsaufnahme des zugeordneten Projektionswinkels basieren, beispielsweise unter Verwendung dieser Projektionsaufnahme in Form einer Durchschnittsintensitätsprojektion.

In einer Ausgestaltung können mehrere (erste) synthetische Mammogramme erzeugt werden. Die maximale Anzahl der erzeugten (ersten) synthetischen Mammogramme kann beispielsweise der Anzahl der Projektionswinkel entsprechen. Bevorzugt wird ein (erstes) synthetisches Mammogramm für den Projektionswinkel 0 Grad erzeugt. Weiterhin werden 2 bis zur maximalen Anzahl der aufgenommenen Projektionswinkel, bevorzugt 10 bis 20, besonders bevorzugt 17, (erste) synthetische Mammogramme erzeugt. Beispielsweise werden 17 (erste) synthetische Mammogramme basierend auf 25 Projektionsdatensätzen erzeugt.

Es kann eine Durchschnittsintensitätsprojektion (AIP) basierend auf der Mehrzahl von Projektionsdatensätzen als erste Bildkomponente ermittelt werden. Bevorzugt kann ein Projektionsdatensatz eines Projektionswinkels, insbesondere entsprechend der Zuordnung eines Projektionswinkels zum (ersten) synthetischen Mammogramm, als Durchschnittsintensitätsprojektion oder als Basis für die Durchschnittsintensitätsprojektion verwendet werden. Insbesondere kann eine limited-angle-AIP (LARIP) als Durchschnittsintensitätsprojektion verwendet werden. Es kann ein Projektionsdatensatz oder es können mehrere Projektionsdatensätze ausgewählt werden, auf deren Basis die Durchschnittsintensitätsprojektion ermittelt wird. Vorteilhaft umfasst die Durchschnittsintensitätsprojektion als erste Bildkomponente wesentliche Informationen über das Untersuchungsobjekt in der zweidimensionalen Draufsicht entsprechend des Projektionswinkels. Die Durchschnittsintensitätsprojektion kann allein jedoch kaum den Anforderungen für eine umfassende Beurteilung der Brust genügen, da sie aufgrund der nur anteilig verwendeten Dosis ein relativ hohes Rauschen aufweist.

Auf die Durchschnittsintensitätsprojektion können zumindest einer der folgenden Schritte angewendet werden: Intensitätsanpassung, Grauwertverteilungsanpassung, Streustrahlenkorrektur, und kalkerhaltender Rauschfilter. Die Durchschnittsintensitätsprojektion kann derart bearbeitet werden, dass die bearbeitete Durchschnittsintensitätsprojektion als rauschige Basis bzw. erste Bildkomponente für das (erste) synthetische Mammogramm verwendet werden kann. Vorteilhaft kann die Information dieser Projektion als Grundlage für das (erste) synthetische Mammogramm dienen. Auf die erste Bildkomponente aufbauend kann durch Hinzufügen von Kanteninformationen und Kontrastinformationen einer zweiten Bildkomponente ein zu einer konventionellen digitalen Vollfeld-Mammographieaufnahme im Wesentlichen qualitativ äquivalentes (erstes) synthetisches Mammogramm erzeugt werden.

Es kann eine Maximumsintensitätsprojektion (MIP) basierend auf der Mehrzahl von Projektionsdatensätzen als zweite Bildkomponente ermittelt werden. Es können insbesondere mehrere Projektionsdatensätze zum Ermitteln der Maximumsintensitätsprojektion verwendet werden. Es können insbesondere Projektionsdatensätze verwendet werden, welche benachbart sind zu dem (ersten) synthetischen Mammogramm zugeordneten Projektionsdatensatz bzw. deren Projektionswinkel. Beispielsweise können Projektionsdatensätze eines geeigneten Winkelbereichs um den zugeordneten Projektionswinkel verwendet werden. Es können insbesondere alle Projektionsdatensätze zum Ermitteln der Maximumsintensitätsprojektion verwendet werden. Vorteilhaft können Kanteninformationen und Kontrastinformationen aus mehreren Projektionsdatensätzen als zweite Bildkomponente genutzt werden, um diese der ersten Bildkomponente hinzuzufügen. Vorteilhaft kann die zweite Bildkomponente zum Entrauschen verwendet werden.

Aus dem Tomosynthesevolumen der Niedrigenergietomosyntheseaufnahme kann eine gewichtete Intensitätsprojektion, beispielsweise basierend auf der dreidimensionalen Wahrscheinlichkeitskarte statt einer MIP alternativ als zweite Bildkomponente verwendet werden.

Die erste Bildkomponente und die zweite Bildkomponente können zum (ersten) synthetischen Mammogramm rekombiniert werden. Durch Zusammenfügen bzw. Rekombinieren der ersten Bildkomponente und der zweiten Bildkomponente kann das (erste) synthetische Mammogramm erzeugt werden. Vorteilhaft wird besonders betont die Information des zugeordneten Projektionsdatensatzes in Form der ersten Bildkomponente gemeinsam mit zusätzlichen Informationen aus mehreren bzw. weiteren Projektionsdatensätzen in Form der zweiten Bildkomponente genutzt. Dadurch kann die Information basierend auf der verwendeten Patientendosis vorteilhaft für das Erzeugen des (ersten) synthetischen Mammogramms verwendet werden.

Gemäß einem Aspekt der Erfindung wird im Schritt des Erzeugens durch Vorwärtsprojektion der dreidimensionalen Wahrscheinlichkeitskarte eine zweidimensionale Wahrscheinlichkeitskarte erstellt. Die dreidimensionale Wahrscheinlichkeitskarte mit den Wichtungsfaktoren als Werte für die Voxel der dreidimensionalen Wahrscheinlichkeitskarte können vorwärtsprojiziert werden. Dadurch wird eine zweidimensionale Wahrscheinlichkeitskarte erzeugt. Die zweidimensionale Wahrscheinlichkeitskarte kann bevorzugt Wichtungsfaktoren im Wertebereich zwischen 0 und 2 aufweisen. Der Wert 1 bedeutet keine Verstärkung. Der Wert 0 bedeutet eine vollständige Unterdrückung. Der Wert 2 bedeutet eine maximale Verstärkung.

Die Vorwärtsprojektion der dreidimensionalen Wahrscheinlichkeitskarte kann vorteilhaft für ein synthetisches, insbesondere kontrastverstärktes, Mammogramm genutzt werden. Die Gewichtungsfaktoren in der zwei- bzw. dreidimensionalen Wahrscheinlichkeitskarte kann abhängig von der Brustdicke oder der Röhrenspannung(en) sein.

Gemäß einem Aspekt der Erfindung ist das synthetische Mammogramm eine Überlagerung des ersten synthetischen Mammogramms mit der zweidimensionalen Wahrscheinlichkeitskarte. Im synthetischen Mammogramm kann die zweidimensionale Wahrscheinlichkeitskarte als beispielsweise farbige Überlagerung dargestellt werden. Vorteilhaft können Bereich mit, insbesondere überlagerten, tumorbehafteten Bereichen hervorgehoben werden. Vorteilhaft kann die Diagnose verbessert werden.

Gemäß einem Aspekt der Erfindung basiert das synthetische Mammogramm auf einer Gewichtung des ersten synthetischen Mammogramms mit der zweidimensionalen Wahrscheinlichkeitskarte. Das erste synthetische Mammogramm kann basierend auf der Durchschnittsintensitätsprojektion und der Maximumsintensitätsprojektion erzeugt werden. Das erste synthetische Mammogramm kann mit der zweidimensionalen Wahrscheinlichkeitskarte gewichtet werden. Damit kann ein gewichtetes synthetisches Mammogramm erhalten werden.

Die zweidimensionale Wahrscheinlichkeitskarte, bevorzugt mit Werten zwischen 0 und 2, kann mit dem ersten synthetischen Mammogramm zum synthetischen Mammogramm kombinieren werden. Die Wichtungsfaktoren können pixelweise auf die Bildwerte des ersten synthetischen Mammogramms angewendet werden. Damit wird eine Gewichtung im zweidimensionalen Bildraum vorgenommen. Vorteilhaft kann ein optimiertes synthetisches Mammogramm ermöglicht werden. Vorteilhaft kann eine reduzierte Überlagerung tumorbehafteter Bereiche im synthetischen Mammogramm ermöglicht werden. Vorteilhaft kann die Überlagerung mittels Auswertung nur der zur Untersuchung bzw. Aufnahme gehörigen Daten erfolgen.

Gemäß einem Aspekt der Erfindung wird im Schritt des Erzeugens die dreidimensionale Wahrscheinlichkeitskarte mit der Niedrigenergietomosyntheseaufnahme zu einer gewichteten Niedrigenergietomosyntheseaufnahme kombiniert und die gewichtete Niedrigenergietomosyntheseaufnahme vorwärtsprojiziert. Die Voxel, insbesondere jedes Voxel, des Tomosynthesevolumens basierend auf der Niedrigenergietomosyntheseaufnahme kann mit dem Wichtungsfaktor bzw. Wahrscheinlichkeitsfaktor der dreidimensionalen Wahrscheinlichkeitsfaktor der dreidimensionalen Wahrscheinlichkeitskarte multipliziert bzw. gewichtet werden.

Der Wertebereich der dreidimensionalen Wahrscheinlichkeitskarte kann bevorzugt dem Bereich zwischen 0 und 2 entsprechen. Vorteilhaft kann die Gewichtung im dreidimensionalen Bildraum durchgeführt werden. Diese gewichtete dreidimensionale Bildinformation kann mittels der Vorwärtsprojektion in den zweidimensionalen Bildraum überführt werden.

Gemäß einem Aspekt der Erfindung wird die vorwärtsprojizierte gewichtete Niedrigenergietomosyntheseaufnahme als synthetisches Mammogramm angezeigt. Gemäß einem Aspekt der Erfindung ist die vorwärtsprojizierte gewichtete Niedrigenergietomosyntheseaufnahme das synthetische Mammogramm. Vorteilhaft kann eine übersichtliche Darstellung im zweidimensionalen Bildraum zur Diagnose, insbesondere als Übersichtsbild, genutzt werden.

Gemäß einem Aspekt der Erfindung wird die vorwärtsprojizierte gewichtete Niedrigenergietomosyntheseaufnahme mit einer Durchschnittsintensitätsprojektion zum synthetischen Mammogramm kombiniert. Die Durchschnittsintensitätsprojektion kann beispielsweise eine mittlere Projektion, beispielsweise 0 Grad, der Niedrigenergietomosyntheseaufnahme sein. Die vorwärtsprojizierte gewichtete Niedrigenergietomosyntheseaufnahme umfasst eine gewichtete Intensität pro Pixel. Es kann zusätzlich eine sogenannte Kalk-Maximumsintensitätsprojektion bei der Kombination zum synthetischen Mammogramm berücksichtigt werden. Vorteilhaft kann ein gewichtetes synthetisches Mammogramm ermöglicht werden. Vorteilhaft kann eine Gewichtung im dreidimensionalen Bildraum zu einer besonders starken Reduktion von Überlagerungen tumorbehafteter Bereich führen.

Gemäß einem Aspekt der Erfindung ist das synthetische Mammogramm eine Überlagerung der vorwärtsprojizierte gewichtete Niedrigenergietomosyntheseaufnahme mit einem ersten synthetischen Mammogramm basierend auf der Niedrigenergietomosyntheseaufnahme. Im synthetischen Mammogramm kann die zweidimensionale vorwärtsprojizierte gewichtete Niedrigenergietomosyntheseaufnahme als beispielsweise farbige Überlagerung über dem ersten synthetischen Mammogramm dargestellt werden. Vorteilhaft können Bereiche mit, insbesondere überlagerten, tumorbehafteten Bereichen hervorgehoben werden. Vorteilhaft kann die Diagnose verbessert werden. Vorteilhaft kann ein übliches erstes synthetisches Mammogramm mit der Überlagerung basierend auf der dreidimensionalen Gewichtung dargestellt werden.

Gemäß einem Aspekt der Erfindung wird die Summe der Wichtungsfaktoren der dreidimensionalen Wahrscheinlichkeitskarte für einen Pixel des synthetischen Mammogramms beitragenden, insbesondere allen, Schichten auf einen vorbestimmten Wert normiert. Die Normierung einander zugeordneter Voxel der mehreren Schichten kann 1 bzw. 100 betragen. Die zugeordneten Voxel können derart definiert werden, dass diese Voxel zu einem gemeinsamen Pixel im synthetischen Mammogramm beitragen. Vorteilhaft kann eine einheitliche Darstellung von verschiedenen synthetischen Mammogrammen, beispielsweise basierend auf unterschiedlichen Untersuchungen, ermöglicht werden.

Erfindungsgemäß basieren die Wichtungsfaktoren der dreidimensionalen Wahrscheinlichkeitskarte auf einer quantitativen Analyse der Kontrastmittelkonzentration. Das Kontrastmittel kann insbesondere Jod sein. Die Kontrastmittelkonzentration kann in mg Jod/cm$^3$ angegeben werden.

Gemäß einem Aspekt der Erfindung wird die dreidimensionale Wahrscheinlichkeitskarte in einem Volumenbild, insbesondere zusätzlich zum synthetischen Mammogramm, basierend auf der Niedrigenergietomosyntheseaufnahme und/oder der Hochenergietomosyntheseaufnahme überlagert angezeigt. Beispielsweise kann ein Scrollen bzw. Blättern bzw. Bewegen der Ansicht des Volumenbilds in Form des Subtraktionsvolumens ermöglicht werden. Bevorzugt kann eine interessierende Region (ROI) im synthetischen Mammogramm ausgewählt und ggf. markiert werden. Es kann eine automatische Anzeige des Volumenbilds bzw. der entsprechenden Schicht des Volumenbilds basierend auf der Auswahl der interessierenden Region erfolgen. Vorteilhaft können die Übersichtlichkeit des zweidimensionalen synthetischen Mammogramms mit der Tiefenauflösung des dreidimensionalen Volumenbilds kombiniert werden. Vorteilhaft kann eine verbesserte Diagnose ermöglicht werden.

Die Erfindung betrifft ferner ein Mammographiesystem aufweisend Mittel zum Durchführen eines erfindungsgemäßen Verfahrens. Die Vorteile des erfindungsgemäßen Verfahrens können vorteilhaft auf das Mammographiesystem übertragen werden. Das Mammographiesystem kann folgende Einheiten umfassen:

- eine Aufnahmeeinheit zum Aufnehmen einer Niedrigenergietomosyntheseaufnahme mit einem ersten Röntgenenergiespektrum und zum Aufnehmen einer Hochenergietomosyntheseaufnahme mit einem zweiten gegenüber dem ersten Röntgenenergiespektrum höherenergetischen Röntgenenergiespektrum, wobei der Untersuchungsbereich eine Kontrastmittelverteilung aufweist,
- eine Ermittlungseinheit zum Ermitteln eines Subtraktionsvolumens basierend auf der Hochenergietomosyntheseaufnahme und der Niedrigenergietomosyntheseaufnahme,
- eine Erstellungseinheit zum Erstellen einer dreidimensionalen Wahrscheinlichkeitskarte mit einem Wichtungsfaktor pro Voxel basierend auf dem Subtraktionsvolumen,
- eine Erzeugungseinheit zum Erzeugen eines synthetischen Mammogramms basierend auf der dreidimensionalen Wahrscheinlichkeitskarte, und
- eine Anzeigeeinheit, beispielsweise in Form eines Bildschirms, zum Anzeigen des synthetischen Mammogramms.

Die Ermittlungseinheit, die Erstellungseinheit und die Erzeugungseinheit können von einer Rechnereinheit des Mammographiesystems umfasst sein. Die Aufnahmeeinheit kann insbesondere die Röntgenquelle und den Röntgendetektor umfassen.

Die Erfindung betrifft ferner ein Computerprogrammprodukt mit einem Computerprogramm, welches direkt in eine Speichereinrichtung einer Steuereinrichtung eines Mammographiesystems ladbar ist, mit Programmabschnitten, um alle Schritte eines erfindungsgemäßen Verfahrens auszuführen, wenn das Computerprogramm in der Steuereinrichtung des Mammographiesystems ausgeführt wird.

Die Erfindung betrifft ferner ein computerlesbares Medium, auf welchem von einer Rechnereinheit einlesbare und ausführbare Programmabschnitte gespeichert sind, um alle Schritte eines erfindungsgemäßen Verfahrens auszuführen, wenn die Programmabschnitte von dem Mammographiesystem bzw. dessen Rechnereinheit ausgeführt werden.

Nachfolgend werden Ausführungsbeispiele der Erfindung anhand von Zeichnungen näher erläutert. Hierbei zeigt:

FIG 1 eine schematische Darstellung des erfindungsgemäßen Verfahrens;

FIG 2 eine schematische Darstellung des erfindungsgemäßen Mammographiesystems in einer ersten Ausführungsform; und

FIG 3 eine schematische Darstellung des erfindungsgemäßen Mammographiesystems in einer zweiten Ausführungsform.

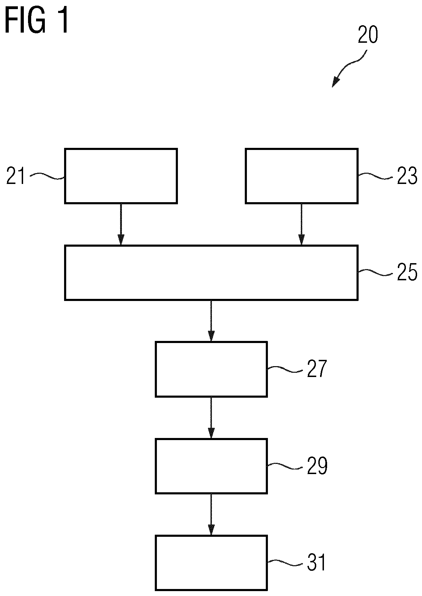

Die Fig. 1 zeigt eine beispielhafte Ausführung des erfindungsgemäßen Verfahrens 20 zum Erzeugen eines synthetischen Mammogramms basierend auf einer Dual-Energy-Tomosyntheseaufnahme eines Untersuchungsbereichs. Das Verfahren 20 weist die folgenden Schritte auf: Im Schritt des Aufnehmens 21 wird eine Niedrigenergietomosyntheseaufnahme mit einem ersten Röntgenenergiespektrum aufgenommen. Im Schritt des Aufnehmens 23 wird eine Hochenergietomosyntheseaufnahme mit einem zweiten gegenüber dem ersten Röntgenenergiespektrum höherenergetischen Röntgenenergiespektrum aufgenommen, wobei der Untersuchungsbereich eine Kontrastmittelverteilung aufweist. Im Schritt des Ermittelns 25 wird ein Subtraktionsvolumen basierend auf der Hochenergietomosyntheseaufnahme und der Niedrigenergietomosyntheseaufnahme ermittelt. Im Schritt des Erstellens 27 wird eine dreidimensionale Wahrscheinlichkeitskarte mit einem Wichtungsfaktor pro Voxel basierend auf dem Subtraktionsvolumen erstellt. Im Schritt des Erzeugens 29 wird ein synthetischen Mammogramm basierend auf der dreidimensionalen Wahrscheinlichkeitskarte erzeugt. Es kann sich ein Schritt des Anzeigens 31 des synthetischen Mammogramms anschließen.

Im Schritt des Erzeugens 29 kann ein erstes synthetisches Mammogramm basierend auf der Niedrigenergietomosyntheseaufnahme erstellt werden. Im Schritt des Erzeugens 29 kann ferner durch eine Vorwärtsprojektion der dreidimensionalen Wahrscheinlichkeitskarte eine zweidimensionale Wahrscheinlichkeitskarte erstellt werden. Das synthetische Mammogramm kann eine Überlagerung des ersten synthetischen Mammogramms mit der zweidimensionalen Wahrscheinlichkeitskarte sein. Alternativ kann das synthetische Mammogramm auf einer Gewichtung des ersten synthetischen Mammogramms mit der zweidimensionalen Wahrscheinlichkeitskarte basieren.

In einer alternativen Ausgestaltung kann im Schritt des Erzeugens 29 die dreidimensionale Wahrscheinlichkeitskarte mit der Niedrigenergietomosyntheseaufnahme zu einer gewichteten Niedrigenergietomosyntheseaufnahme kombiniert werden und die gewichteten Niedrigenergietomosyntheseaufnahme vorwärtsprojiziert werden. Die vorwärtsprojizierte gewichtete Niedrigenergietomosyntheseaufnahme kann das synthetisches Mammogramm sein. Alternativ kann die vorwärtsprojizierte gewichtete Niedrigenergietomosyntheseaufnahme mit einer Durchschnittsintensitätsprojektion zum synthetischen Mammogramm kombiniert werden. Alternativ kann das synthetische Mammogramm eine Überlagerung der vorwärtsprojizierte gewichtete Niedrigenergietomosyntheseaufnahme mit einem ersten synthetischen Mammogramm basierend auf der Niedrigenergietomosyntheseaufnahme sein.

Im Schritt des Anzeigens 31 kann ferner die dreidimensionale Wahrscheinlichkeitskarte in einem Volumenbild, insbesondere dem Subtraktionsvolumenbild, basierend auf der Niedrigenergietomosyntheseaufnahme und/oder der Hochenergietomosyntheseaufnahme überlagert angezeigt werden.

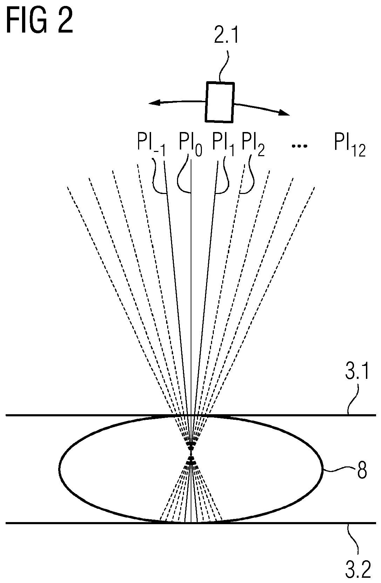

Die Fig. 2 zeigt eine beispielhafte Ausführung des erfindungsgemäßen Mammographiesystems in einer ersten Ausführungsform. Es wird für eine Niedrigenergietomosyntheseaufnahme und eine Hochenergietomosyntheseaufnahme jeweils eine Mehrzahl von Projektionsdatensätzen unter einer Mehrzahl von Projektionswinkeln $PI_{-1,0,1,2,...,12}$ aufgenommen. Die Röntgenquelle 2.1 wird dabei insbesondere entlang eines Radius um einen Punkt in der Brust 8 verfahren, wobei unter den Projektionswinkeln $PI_{-1,0,1,2,..,12}$ jeweils ein Projektionsdatensatz aufgenommen wird. Während der Aufnahme ist die Brust 8 eines Patienten als Untersuchungsobjekt zwischen einem oberen Kompressionselement 3.1 und einem unteren Kompressionselement 3.2 angeordnet.

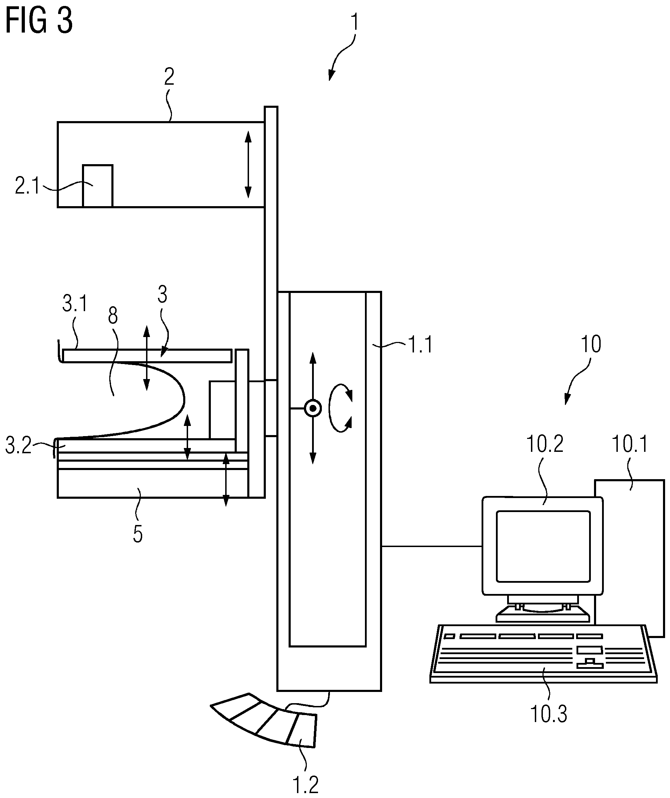

Die Fig. 3 zeigt eine beispielhafte Ausführung des erfindungsgemäßen Mammographiesystems 1 in einer zweiten Ausführungsform. Das Mammographiesystem 1 umfasst ein Stativ 1.1, an welchem das Röntgengehäuse 2 aufweisend die Röntgenquelle 2.1 und der Röntgendetektor 5 gemeinsam mit einer Kompressionseinheit 3 angeordnet sind. Insbesondere das Röntgengehäuse 2 ist bezüglich des Stativs 1.1 und des Röntgendetektors 5 sowie der Kompressionseinheit 3 rotierbar am Stativ 1.1 gelagert. Die Kompressionseinheit 3 umfasst ein oberes Kompressionselement 3.1 und ein unteres Kompressionselement 3.2, zwischen denen die Brust 8 eines Patienten angeordnet wird. Das Mammographiesystem 1 ist mit einer Datenverarbeitungseinheit 10 verbunden. Die Datenverarbeitungseinheit 10 umfasst mindestens eine Prozessoreinheit bzw. Rechnereinheit 10.1, eine Anzeigeeinheit 10.2 und eine Eingabeeinheit 10.3. Über die Eingabeeinheit kann eine Markierung der interessierenden Region erfolgen. Die Rechnereinheit 10.1 kann die Ermittlungseinheit, die Erstellungseinheit und die Erzeugungseinheit umfassen. Das Mammographiesystem 1 ist zumindest teilweise über einen Fußschalter 1.2 steuerbar.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Verfahren (20) zum Erzeugen eines synthetischen Mammogramms basierend auf einer Dual-Energy-Tomosyntheseaufnahme eines Untersuchungsbereichs aufweisend die Schritte:

   a. Aufnehmen (21) einer Niedrigenergietomosyntheseaufnahme mit einem ersten Röntgenenergiespektrum,

   b. Aufnehmen (23) einer Hochenergietomosyntheseaufnahme mit einem zweiten gegenüber dem ersten Röntgenenergiespektrum höherenergetischen Röntgenenergiespektrum, wobei der Untersuchungsbereich eine Kontrastmittelverteilung aufweist,

   c. Ermitteln (25) eines Subtraktionsvolumens basierend auf der Hochenergietomosyntheseaufnahme und der Niedrigenergietomosyntheseaufnahme,

   d. Erstellen (27) einer dreidimensionalen Wahrscheinlichkeitskarte mit einem Wichtungsfaktor pro Voxel basierend auf dem Subtraktionsvolumen,

   e. Erzeugen (29) eines synthetischen Mammogramms basierend auf der dreidimensionalen Wahrscheinlichkeitskarte,

   **dadurch gekennzeichnet, dass** die Wichtungsfaktoren der dreidimensionalen Wahrscheinlichkeitskarte auf einer quantitativen Analyse der Kontrastmittelkonzentration basieren.

2. Verfahren nach Anspruch 1, wobei im Schritt des Erzeugens ein erstes synthetisches Mammogramm basierend auf der Niedrigenergietomosyntheseaufnahme erstellt wird.

3. Verfahren nach Anspruch 2, wobei im Schritt des Erzeugens durch Vorwärtsprojektion der dreidimensionalen Wahrscheinlichkeitskarte eine zweidimensionale Wahrscheinlichkeitskarte erstellt wird.

4. Verfahren nach Anspruch 3, wobei das synthetische Mammogramm eine Überlagerung des ersten synthetischen Mammogramms mit der zweidimensionalen Wahrscheinlichkeitskarte ist.

5. Verfahren nach Anspruch 3, wobei das synthetische Mammogramm auf einer Gewichtung des ersten synthetischen Mammogramms mit der zweidimensionalen Wahrscheinlichkeitskarte basiert.

6. Verfahren nach Anspruch 1, wobei im Schritt des Erzeugens die dreidimensionale Wahrscheinlichkeitskarte mit der Niedrigenergietomosyntheseaufnahme zu einer gewichteten Niedrigenergietomosyntheseaufnahme kombiniert wird und die gewichtete Niedrigenergietomosyntheseaufnahme vorwärtsprojiziert wird.

7. Verfahren nach Anspruch 6, wobei die vorwärtsprojizierte gewichtete Niedrigenergietomosyntheseaufnahme das synthetische Mammogramm ist.

8. Verfahren nach Anspruch 6, wobei die vorwärtsprojizierte gewichtete Niedrigenergietomosyntheseaufnahme mit einer Durchschnittsintensitätsprojektion zum synthetischen Mammogramm kombiniert wird.

9. Verfahren nach den Ansprüchen 2 und 6, wobei das synthetische Mammogramm eine Überlagerung der vorwärtsprojizierte gewichtete Niedrigenergietomosyntheseaufnahme mit einem ersten synthetischen Mammogramm basierend auf der Niedrigenergietomosyntheseaufnahme ist.

10. Verfahren nach einem der vorangehenden Ansprüche, wobei die Summe der Wichtungsfaktoren der dreidimensionalen Wahrscheinlichkeitskarte für einen Pixel des synthetischen Mammogramms beitragenden Schichten auf einen vorbestimmten Wert normiert wird.

11. Verfahren nach einem der vorangehenden Ansprüche, wobei die dreidimensionale Wahrscheinlichkeitskarte in einem Volumenbild basierend auf der Niedrigenergietomosyntheseaufnahme und/oder der Hochenergietomosyntheseaufnahme überlagert angezeigt wird.

12. Mammographiesystem (1) aufweisend Mittel zum Durchführen eines Verfahrens nach einem der Ansprüche 1 bis 11, aufweisend die Einheiten:

a. eine Aufnahmeeinheit zum Aufnehmen einer Niedrigenergietomosyntheseaufnahme mit einem ersten Röntgenenergiespektrum und zum Aufnehmen einer Hochenergietomosyntheseaufnahme mit einem zweiten gegenüber dem ersten Röntgenenergiespektrum höherenergetischen Röntgenenergiespektrum, wobei der Untersuchungsbereich eine Kontrastmittelverteilung aufweist,
b. eine Ermittlungseinheit zum Ermitteln eines Subtraktionsvolumens basierend auf der Hochenergietomosyntheseaufnahme und der Niedrigenergietomosyntheseaufnahme,
c. eine Erstellungseinheit zum Erstellen einer dreidimensionalen Wahrscheinlichkeitskarte mit einem Wichtungsfaktor pro Voxel basierend auf dem Subtraktionsvolumen,
d. eine Erzeugungseinheit zum Erzeugen eines synthetischen Mammogramms basierend auf der dreidimensionalen Wahrscheinlichkeitskarte, und
e. eine Anzeigeeinheit, beispielsweise in Form eines Bildschirms, zum Anzeigen des synthetischen Mammogramms,

**dadurch gekennzeichnet, dass** die Wichtungsfaktoren der dreidimensionalen Wahrscheinlichkeitskarte auf einer quantitativen Analyse der Kontrastmittelkonzentration basieren.

13. Computerprogrammprodukt mit einem Computerprogramm, welches direkt in eine Speichereinrichtung einer Steuereinrichtung eines Mammographiesystems ladbar ist, mit Programmabschnitten, um alle Schritte eines Verfahrens nach einem der Ansprüche 1 bis 11 auszuführen, wenn das Computerprogramm in der Steuereinrichtung des Mammographiesystems ausgeführt wird.

14. Computerlesbares Medium, auf welchem von einer Rechnereinheit einlesbare und ausführbare Programmabschnitte gespeichert sind, um alle Schritte eines Verfahrens nach einem der Ansprüche 1 bis 11 auszuführen, wenn die Programmabschnitte von dem Mammographiesystem ausgeführt werden.

## Claims

1. Method (20) for creating a synthetic mammogram on the basis of a dual energy tomosynthesis recording of an examination region, having the steps:

a. making (21) a low energy tomosynthesis recording with a first X-ray energy spectrum,

b. making (23) a high energy tomosynthesis recording with a second X-ray energy spectrum of higher energy compared with the first X-ray energy spectrum, wherein the examination region has a contrast medium distribution,
c. determining (25) a subtraction volume on the basis of the high energy tomosynthesis recording and the low energy tomosynthesis recording,
d. generating (27) a three-dimensional probability map with a weighting factor per voxel on the basis of the subtraction volume,
e. creating (29) a synthetic mammogram on the basis of the three-dimensional probability map,

**characterised in that** the weighting factors of the three-dimensional probability map are based upon a quantitative analysis of the contrast medium concentration.

2. Method according to claim 1, wherein in the step of creating, a first synthetic mammogram is generated on the basis of the low energy tomosynthesis recording.

3. Method according to claim 2, wherein in the step of creating, by means of forward projection of the three-dimensional probability map, a two-dimensional probability map is generated.

4. Method according to claim 3, wherein the synthetic mammogram is a superposition of the first synthetic mammogram with the two-dimensional probability map.

5. Method according to claim 3, wherein the synthetic mammogram is based upon a weighting of the first synthetic mammogram with the two-dimensional probability map.

6. Method according to claim 1, wherein in the step of creating, the three-dimensional probability map is combined with the low energy tomosynthesis recording to a weighted low energy tomosynthesis recording and the weighted low energy tomosynthesis recording is forward projected.

7. Method according to claim 6, wherein the forward-projected weighted low energy tomosynthesis recording is the synthetic mammogram.

8. Method according to claim 6, wherein the forward-projected weighted low energy tomosynthesis recording is combined with an average intensity projection to the synthetic mammogram.

9. Method according to claims 2 and 6, wherein the synthetic mammogram is a superposition of the forward-projected weighted low energy tomosynthesis recording with a first synthetic mammogram on the basis of the low energy tomosynthesis recording.

10. Method according to one of the preceding claims, wherein the total of the weighting factors of the three-dimensional probability map is normalised to a predetermined value for slices contributing one pixel of the synthetic mammogram.

11. Method according to one of the preceding claims, wherein the three-dimensional probability map is displayed in a volume image on the basis of the low energy tomosynthesis recording and/or superposed on the high energy tomosynthesis recording.

12. Mammography system (1) having means for carrying out a method according to one of claims 1 to 11, having the units:

   a. a recording unit for making a low energy tomosynthesis recording with a first X-ray energy spectrum and for making a high energy tomosynthesis recording with a second X-ray energy spectrum of higher energy compared with the first X-ray energy spectrum, wherein the examination region has a contrast medium distribution,
   b. a determining unit for determining a subtraction volume on the basis of the high energy tomosynthesis recording and the low energy tomosynthesis recording,
   c. a generating unit for generating a three-dimensional probability map with a weighting factor per voxel on the basis of the subtraction volume,
   d. a creating unit for creating a synthetic mammogram on the basis of the three-dimensional probability map, and
   e. a display unit, for example in the form of a screen, for displaying the synthetic mammogram,

   **characterised in that** the weighting factors of the three-dimensional probability map are based on a quantitative analysis of the contrast agent concentration.

13. Computer program product having a computer program which can be directly loaded into a memory store of a control apparatus of a mammography system, having program portions in order to carry out all the steps of a method according to one of claims 1 to 11 when the computer program is executed in the control apparatus of the mammography system.

14. Computer-readable medium on which program portions that are configured to be read in and executed by a computer unit are stored, in order to carry out all the steps of a method according to one of claims 1 to 11 when the program portions are executed by the mammography system.

## Revendications

1. Procédé (20) de production d'un mammogramme synthétique sur la base d'un enregistrement par tomosynthèse à énergie duale d'une partie à examiner comportant les stades :

   a. enregistrement (21) d'un enregistrement par tomosynthèse à basse énergie par un premier spectre d'énergie à rayons X,
   b. enregistrement (23) d'un enregistrement par tomosynthèse à haute énergie par un deuxième spectre d'énergie à rayons X, d'énergie plus grande que le premier spectre d'énergie à rayons X, dans lequel la partie à examiner a une répartition d'agent de contraste,
   c. détermination (25) d'un volume de soustraction sur la base de l'enregistrement par tomosynthèse à haute énergie et de l'enregistrement par tomosynthèse à basse énergie,
   d. établissement (27) d'une carte de probabilité en trois dimensions avec un facteur de pondération par voxel sur la base du volume de soustraction,
   e. production (29) d'un mammogramme synthétique sur la base de la carte de probabilité en trois dimensions,

   **caractérisé en ce que** les facteurs de pondération de la carte de probabilité en trois dimensions reposent sur une analyse quantitative de la concentration de l'agent de contraste.

2. Procédé suivant la revendication 1, dans lequel dans le stade de la production, on établit un premier mammogramme synthétique sur la base de l'enregistrement par tomosynthèse à basse énergie.

3. Procédé suivant la revendication 2, dans lequel dans le stade de la production, on établit par projection en avant de la carte de probabilité en trois dimensions, une carte de probabilité en deux dimensions.

4. Procédé suivant la revendication 3, dans lequel le mammogramme synthétique est une superposition du premier mammogramme synthétique avec la carte de probabilité en deux dimensions.

5. Procédé suivant la revendication 3, dans lequel le mammogramme synthétique repose sur une pondération du premier mammogramme synthétique par la carte de la probabilité en deux dimensions.

6. Procédé suivant la revendication 1, dans lequel dans le stade de la production, on combine la carte de probabilité en trois dimensions avec l'enregistrement par tomosynthèse à basse énergie en un enregistrement par tomosynthèse à basse énergie pondérée et on projette en avant l'enregistrement par tomosynthèse à basse énergie pondérée.

7. Procédé suivant la revendication 6, dans lequel l'enregistrement par tomosynthèse à basse énergie pondérée projeté en avant est le mammogramme synthétique.

8. Procédé suivant la revendication 6, dans lequel on combine l'enregistrement par tomosynthèse à basse énergie pondérée projeté en avant à une projection d'intensité moyenne en le mammogramme synthétique.

9. Procédé suivant les revendications 2 et 6, dans lequel le mammogramme synthétique est une superposition de l'enregistrement par tomosynthèse à basse énergie pondérée projeté en avant avec un premier mammogramme synthétique reposant sur l'enregistrement par tomosynthèse à basse énergie.

10. Procédé suivant l'une des revendications précédentes, dans lequel on norme à une valeur déterminée à l'avance la somme des facteurs de pondération de la carte de probabilité en trois dimensions pour des couches contribuant à un pixel du mammogramme synthétique.

11. Procédé suivant l'une des revendications précédentes, dans lequel on affiche en superposition la carte de probabilité en trois dimensions dans une image en volume sur la base de l'enregistrement par tomosynthèse à base énergie et/ou de l'enregistrement par tomosynthèse à haute énergie.

12. Système (1) de mammographie comportant des moyens pour effectuer un procédé suivant l'une des revendications 1 à 11, comportant les unités :

   a. une unité d'enregistrement pour l'enregistrement d'un enregistrement par tomosynthèse à basse énergie par un premier spectre d'énergie de rayons X et pour l'enregistrement d'un enregistrement par tomosynthèse à haute énergie par un deuxième spectre d'énergie à rayons X plus énergétique que le premier spectre d'énergie par rayons X, dans lequel la partie à examiner a une répartition d'agent de contraste,
   b. une unité de détermination pour la détermination d'un volume de soustraction sur la base de l'enregistrement par tomosynthèse à haute énergie et de l'enregistrement par tomosynthèse à basse énergie,

c. une unité d'établissement pour l'établissement d'une carte de probabilité en trois dimensions par un facteur de pondération par voxel sur la base du volume de soustraction,
d. une unité de production pour la production d'un mammogramme synthétique sur la base de la carte de probabilité en trois dimensions, et
e. une unité d'affichage, par exemple sous la forme d'un écran, pour l'affichage du mammogramme synthétique,

**caractérisé en ce que** les facteurs de pondération de la carte de probabilité en trois dimensions reposent sur une analyse quantitative de la concentration de l'agent de contraste.

13. Produit de programme d'ordinateur comprenant un programme d'ordinateur, qui peut être chargé directement dans un dispositif de mémoire d'un dispositif de commande d'un système de mammographie, comprenant des parties de programme, pour exécuter tous les stades d'un procédé suivant l'une des revendications 1 à 11, lorsque le programme d'ordinateur est exécuté dans les dispositifs de commande du système de mammographie.

14. Support déchiffrable par ordinateur, sur lequel sont mises en mémoire des parties de programme pouvant être lues et exécutées par une unité informatique pour exécuter tout le stade d'un procédé suivant l'une des revendications 1 à 11, lorsque les parties de programme sont exécutées par le système de mammographie.

FIG 1

FIG 2

FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9808215 B2
- US 2017011534 A1
- EP 3326535 A1
- US 10010302 B2

**In der Beschreibung aufgeführte Nicht-Patentliteratur**

- **HAILIANG HUANG et al.** Comparison of contrast-enhanced digital mammography and contrast-enhanced digital breast tomosynthesis for lesion assessment. *JOURNAL OF MEDICAL IMAGING,* 13. Februar 2019, vol. 6 (03), ISSN 2329-4302, 1